# EUROPÄISCHE PATENTANMELDUNG

(11) **EP 2 500 041 A1**
(43) Veröffentlichungstag der Anmeldung: **19.09.2012**
(21) Anmeldenummer: 12155603.9
(22) Anmeldetag: 15.02.2012
(51) Int. Cl.: A61L 2/18, A61L 2/24, A61C 19/00

(54) **Gerät und Verfahren zum Reinigen, Desinfizieren, Sterilisieren und/oder Pflegen von ärztliche Instrumenten**

(30) Priorität: 18.03.2011 DE 102011005793
(71) Anmelder: Kaltenbach & Voigt GmbH, 88400 Biberach (DE)
(72) Erfinder: Wiek, Hans Dieter, 88454 Hochdorf (DE); Heckenberger, Hans, 88433 Aßmannshardt (DE)
(74) Vertreter: Thun, Clemens

(57) **Zusammenfassung**

Gerät (1) und Verfahren zum Reinigen, Desinfizieren, Sterilisieren und/oder Pflegen von ärztlichen, insbesondere zahnärztlichen Instrumenten (4), wobei die aufzubereitenden Instrumente (4) an Halterungen (3) in einer Pflege- und Reinigungskammer (2) des Geräts (1) angeordnet werden und den Instrumenten (4) durch Mittel (7, 12, 14) zum Zuführen von Reinigungs- oder Pflegemedien ein Reinigungsmedium zur Reinigung zugeführt wird und wobei das Reinigungsmedium durch eine Vorrichtung (9) zur Temperierung auf einen vorgegebenen Temperaturbereich temperiert wird.

## Beschreibung

Die vorliegende Erfindung betrifft ein Gerät und ein Verfahren, welches zum Reinigen, Desinfizieren, Sterilisieren und/oder Pflegen von ärztlichen Instrumenten, insbesondere zahnärztlichen Instrumenten, vorgesehen ist, das eine Pflege- und Reinigungskammer, in der Pflege- und Reinigungskammer befindliche Halterungen zur Aufnahme der Instrumente und Mittel zum Zuführen von Reinigungs- oder Pflegemedien aufweist.

Bei ärztlichen oder zahnärztlichen Instrumenten oder Handstücken handelt es sich häufig um rohrförmige Teile, die der Arzt oder Zahnarzt während der Behandlung als Griffhülse ergreift. Ein üblicherweise in der zahnmedizinischen Praxis verwendetes Instrument ist beispielsweise ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist.

Zur Aufrechterhaltung der Funktion dieser Instrumente bedarf es nun von Zeit zu Zeit zum einen einer Pflege, insbesondere der drehbar gelagerten Antriebselemente. Zum anderen führen die in der zahnärztlichen Praxis immer weiter ansteigenden Hygieneanforderungen dazu, dass eine Aufbereitung von derartigen Instrumenten in regelmäßigen und häufigen Abständen erforderlich ist.

Hierzu werden die Instrumente einer Reinigung, Desinfektion, Sterilisation und/oder Pflege unterzogen. Bei der Reinigung ist beispielsweise vorgesehen, dass die Handstücke bzw. Instrumente mit einem Reinigungsmedium gereinigt werden, um Rückstände wie Blutreste, Speichel und andere organische Verschmutzungen zu entfernen. Im Einzelnen werden diese zur Reinigung als erstes mit dem Reinigungsmedium gespült. Anschließend wird einige Zeit gewartet, damit das Reinigungsmedium entsprechend einwirken kann. Nach Ablauf der Wartezeit wird das Reinigungsmedium dann mit Hilfe von Druckluft aus dem Instrument ausgeblasen, wobei nach dem Ausblasen kurz gewartet wird, um den im Instrument entstehenden Druck abzubauen. Diese Vorgehendweise kann nun mehrmals wiederholt werden, um eine möglichst gute Reinigung der Handstücke bzw. Instrumente zu erreichen.

Nachdem die Instrumente in einem ersten Schritt gereinigt worden sind, ist desweiteren dann zur Desinfektion bzw. Sterilisation vorgesehen, dass die Instrumente mit Dampf durchblasen werden. Hierfür wird das Reinigungsmedium impulsweise in einen Verdampfer gepumpt. Das dort verdampfte Reinigungsmedium wird dann als Dampf durch die Instrumente geleitet und spült dabei beispielsweise ölhaltige Verschmutzungen aus. Auch hier wird wieder einige Zeit gewartet, um dem Dampf ein Einwirken zu ermöglichen, wodurch auch eine Keimreduktion erfolgt. Zur Pflege der Instrumente werden diese im Anschluss daran mit Druckluft durchblasen, wobei der Druckluft Öl dosiert zugegeben wird, das durch den Druckluftstrom in den Instrumenten verteilt wird. Bei diesem Vorgang werden die Instrumente bereits etwas abgekühlt.

In einem letzten Schritt ist danach vorgesehen, dass die Instrumente mit normaler Druckluft durchblasen werden, wodurch zum einen erreicht wird, dass die Instrumente unter 50° abgekühlt werden, und zum anderen überschüssiges Öl aus den Instrumenten entfernt wird.

Die vorliegende Erfindung bezieht sich nun insbesondere aber nicht ausschließlich auf den Vorgang der Reinigung der Instrumente. Bisher wird hier als Reinigungsmedium beispielsweise kaltes Wasser verwendet, um Blut und weitere Rückstände zu entfernen.

Der vorliegenden Erfindung liegt nun die Aufgabe zugrunde, eine Gerät und ein Verfahren zum Reinigen, Desinfizieren, Sterilisieren und/oder Pflegen von ärztlichen, insbesondere zahnärztlichen Instrumenten weiter zu verbessern, derart, dass die Reinigung der Instrumente noch effektiver und gründlicher vollzogen wird.

Anstatt kaltem Wasser als Reinigungsmedium zur Reinigung kann nun eine Reinigungslösung vorgesehen sein, die aus demineralisiertem Wasser und einem Reinigungszusatz (beispielsweise positive Validierung mit Helizyme 1%) besteht. Bei einem derartigen Reinigungsmedium auf Enzymbasis ist es nun besonders vorteilhaft, wenn das Reinigungsmedium auf eine Temperatur zwischen 40° und 60° aufgeheizt wird, da mit zunehmender Temperatur die Reaktion schneller abläuft, auf der anderen Seite jedoch bei zu hoher Temperatur die Reinigung nicht mehr optimal abläuft, da beispielsweise Blut bei zu hoher Temperatur verklumpen bzw. verkrusten kann.

Erfindungsgemäß ist daher nun ein Gerät und ein Verfahren zum Reinigen, Desinfizieren, Sterilisieren und/oder Pflegen von ärztlichen, insbesondere zahnärztlichen Instrumenten vorgesehen, bei denen Instrumente an Halterungen in einer Pflege- und Reinigungskammer angeordnet sind und mit Hilfe von Mitteln zum Zuführen von Reinigungs- oder Pflegemedien, den Instrumenten ein Reinigungsmedium zur Reinigung zugeführt wird. Hierbei ist nun insbesondere vorgesehen, dass das Reinigungsmedium durch eine Vorrichtung zur Temperierung auf einen vorgegebenen Temperaturbereich temperiert wird.

Vorteilhafterweise kann vorgesehen sein, dass die Halterungen zur Aufnahme der Instrumente mit einem Kühl-/Heizkreislauf verbunden sind, der thermisch mit den Mitteln zum Zuführen gekoppelt ist, wodurch bei Kühlung der Halterungen, die in den Kühl-/Heizkreislauf abgegebene Wärme zur Temperierung des Reinigungsmediums verwendet werden kann. Hierbei kann der Kühl-/Heizkreislauf mit der Vorrichtung zur Temperierung verbunden sein und mit den Mitteln zum Zuführen in der Vorrichtung zur Temperierung thermisch gekoppelt sein. Die thermische Koppelung des Kühl-/Heizkreislauf und der Mittel zum Zuführen kann durch einen Metallblock erfolgen, der in Lage ist Wärme zu speichern.

Durch den Kühl-/Heizkreislauf ist es möglich, die Halterungen zur Aufnahme der Instrumente nicht nur zu kühlen sondern auch zu erwärmen. Hierzu und zur Temperierung des Reinigungsmediums können in der Vorrichtung zur Temperierung zusätzlich noch Mittel zum Heizen bzw. zum Kühlen vorgesehen sein.

Desweiteren können die Mittel zum Zuführen eines Reinigungsmediums und der Kühl-/Heizkreislauf jeweils eine Pumpe aufweisen.

Vorzugsweise bestehen die Halterungen zur Aufnahme der Instrumente jeweils aus einem Magnetventilblock und einem Kupplungsadapter, wobei die Instrumente an den Kupplungsadaptern angebracht werden.

Durch die vorliegende Erfindung ist es nunmehr möglich, dass ein Reinigungsmedium derart temperiert wird, dass es innerhalb eines bestimmten vorgegebenen Temperaturbereichs, beispielsweise 40-60°, liegt. Durch die zusätzliche Verwendung eines Kühl-/Heizkreislaufs ist es außerdem möglich, dass die beim Desinfizieren entstehende Wärme an den Halterungen zur Erwärmung des Reinigungsmediums, aufgrund der thermischen Kopplung, verwendet werden kann, wobei dies insbesondere dann zum Tragen kommt wenn eine größere Anzahl an Instrumenten gereinigt, desinfiziert, sterilisiert und/oder gepflegt werden soll, sodass mehrere Durchgänge nötig sind um alle Instrumente zu Reinigen, Desinfizieren, Sterilisieren und/oder Pflegen, wodurch dann die beim Desinfizieren entstehende Wärme bei einem ersten Durchgang gespeichert werden kann und anschließend zur Erwärmung eines Reinigungsmediums in einem zweiten Durchgang verwendet werden kann.

Nachfolgend soll die Erfindung anhand der beiliegenden Zeichnungen näher erläutert werden. Es zeigen:
- Figur 1: Gerät zum Reinigen, Desinfizieren, Sterilisieren und/oder Pflegen von ärztlichen, insbesondere zahnärztlichen Instrumenten;
- Figur 2: schematischer Aufbau der Vorrichtung zur Temperierung des Reinigungsmediums, des Kühl-/Heizkreislaufs und der Halterungen zur Aufnahme der Instrumente.

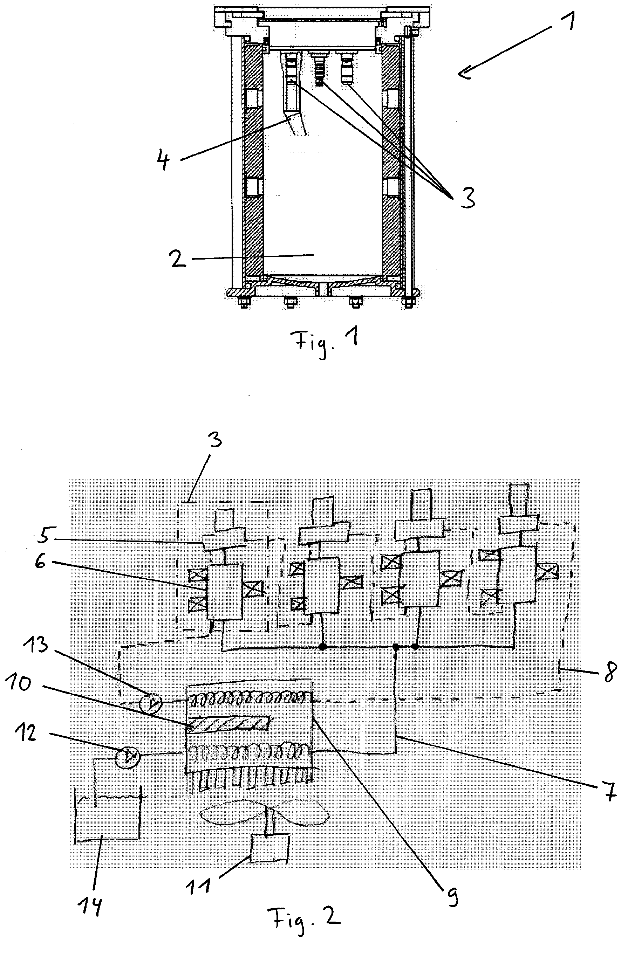

Figur 1 zeigt nun grundsätzlich ein Gerät 1 zum Reinigen, Desinfizieren, Sterilisieren und/oder Pflegen von ärztlichen, insbesondere zahnärztlichen Instrumenten 4, dass eine Pflege- und Reinigungskammer 2 aufweist, in der sich Halterungen 3 zur Aufnahme der Instrumente 4 befinden. Derartige Geräte sind bereits bekannt, weshalb hier auf genauere Erläuterungen zum Aufbau des Geräts 1 verzichtet wird. Auch die bereits zuvor beschriebene Vorgehensweise zum Desinfizieren bzw. Sterilisieren und zum Pflegen der Instrumente 4 sowie die hierfür notwendigen Mittel, werden hier nicht weiter erläutert, da auch diese bereits bekannt sind und für die vorliegende Erfindung ohne Bedeutung sind.

Figur 2 zeigt nun den schematischen Aufbau der erfindungsgemäßen Lösung. Im Einzelnen sind dort nun wiederum die Halterungen 3 zur Aufnahme der Instrumente 4 gezeigt. Diese bestehen wie aus Figur 2 ersichtlich aus einem Kupplungsadapter 5, an dem die Instrumente 4 angebracht werden, und einem Magnetventilblock 6, an dem unter anderem eine Zuführung 7 eines Reinigungsmediums angeschlossen ist und der entsprechend die Zufuhr des Reinigungsmediums steuert.

Desweiteren ist eine Vorrichtung 9 zur Temperierung vorgesehen, durch die die Zuführung 7 des Reinigungsmediums hindurchführt, wodurch das Reinigungsmedium entsprechend temperiert werden kann. Zur Versorgung der Instrumente 4 mit dem Reinigungsmedium ist desweiteren noch ein Vorratsbehälter 14, der beispielsweise ein enzymatisches Reinigungsmedium beinhaltet, und eine Pumpe 12, die das Reinigungsmedium in Richtung der Instrumente 4 pumpt, vorgesehen, die jeweils an die Zuführung 7 des Reinigungsmediums angeschlossen sind.

Durch die Vorrichtung 9 zur Temperierung verläuft zusätzlich noch ein Kühl-/Heizkreislauf 8, derart, dass die Vorrichtung 9 zur Temperierung bzw. die Zuführung 7 des Reinigungsmediums die sich innerhalb der Vorrichtung 9 zur Temperierung befindet thermisch mit dem Kühl-/Heizkreislauf 8 gekoppelt ist. Zur Kühlung bzw. Erwärmung der Halterungen 3 ist der Kühl-/Heizkreislauf 8 des Weiteren mit den Halterungen 3 bzw. mit den Kupplungsadaptern 5 und den Magnetventilblöcken 6 verbunden, wobei auch in dem Kühl-/Heizkreislauf 8 eine Pumpe 13 angeordnet ist.

In der Vorrichtung 9 zur Temperierung, die unter anderem beispielsweise aus einem Metallblock bestehen kann, sind außerdem noch ein Heizelement 10 zum Erwärmen und ein Lüfter 11 zum Kühlen vorgesehen. Der Metallblock, der sowohl mit der Zuführung 7 des Reinigungsmediums als auch mit dem Kühl-/Heizkreislauf 8 thermisch in Kontakt steht, ermöglicht hierbei nun die Speicherung von Wärme in der Vorrichtung 9 zur Temperierung.

Durch diesen in Figur 2 gezeigten Aufbau, der beispielsweise innerhalb eines Gehäuses des erfindungsgemäßen Geräts 1 zum Reinigen, Desinfizieren, Sterilisieren und/oder Pflegen aber auch außerhalb eines derartigen Gehäuses vorgesehen sein kann, ist es nunmehr zum Einen möglich, dass ein Reinigungsmedium auf einen vorgegebenen Temperaturbereich temperiert werden kann. Zum Anderen besteht aber auch die Möglichkeit, mit Hilfe des Kühl-/Heizkreislaufs 8 die Halterungen 3 zur Aufnahme der Instrumente 4 zu kühlen oder zu erwärmen, wobei die beim Kühlen durch den Kühl-/Heizkreislauf 8 abgeführte Wärme in der Vorrichtung 9 zur Temperierung bzw. in dem Metallblock gespeichert und dann zur Erwärmung des Reinigungsmediums verwendet werden kann.

Zusätzlich ist es mit Hilfe des Heizelements 10 auch möglich, dass die Vorrichtung 9 zur Temperierung selber Wärme erzeugt, die das Reinigungsmedium entsprechend erwärmt.

Wie bereits weiter oben ausführlich dargelegt, erfolgt zu Beginn der Aufbereitung der Instrumente 4 als erstes immer die Reinigung der Instrumente 4 und im Anschluss daran erst die Desinfektion bzw. Sterilisation und/oder Pflege der Instrumente 4. Dementsprechend hat der Kühl-/Heizkreislauf 8 noch keine Wärme, die insbesondere beim Desinfizieren der Instrumente 4 entsteht, von den Halterungen 3 zu der Vorrichtung 9 zur Temperierung geleitet. Somit ist es insbesondere hier notwendig, dass das Heizelement 10 das Reinigungsmedium in den entsprechenden Temperaturbereich aufheizt. Gleichzeitig ist es aber auch möglich, dass das Heizelement 10 den Kühl-/Heizkreislauf 8 erwärmt, der dann wiederum den Kupplungsadapter 5 und den Magnetventilblock 6 der Halterungen 3 ebenfalls auf den für das Reinigungsmedium optimalen Temperaturbereich aufheizt, wodurch gewährleistet ist, dass das Reinigungsmedium die Instrumente 4 mit der optimalen Temperatur erreicht.

Im Anschluss an die Reinigung werden die Instrumente 4 durch Dampf desinfiziert, wobei sich hierdurch der Magnetventilblock 6 und der Kupplungsadapter 5 auf über 80° erhitzen. Nach der Desinfektionsphase ist es dementsprechend erforderlich, dass diese gekühlt werden. Dies erfolgt dann durch den Kühl-/Heizkreislauf 8, wobei die abgeführte Wärme in der Vorrichtung 9 zur Temperierung bzw. im Metallblock gespeichert werden kann und falls dies als Kühlung nicht ausreicht, mithilfe des Ventilators bzw. Lüfters 11 eine weitere Abkühlung erfolgt. Zuletzt werden dann die Instrumente 4 noch mit Druckluft und Öl gepflegt und weiter abgekühlt.

Die in der Vorrichtung 9 zur Temperierung bzw. in dem Metallblock gespeicherte Wärme kann dann zur Reinigung neuer Instrumente 4, die im Anschluss an die Reinigung, Desinfektion und Pflege der ersten Instrumente 4 in das Gerät 1 zum Reinigen, Desinfizieren, Sterilisieren und/oder Pflegen eingebracht werden verwendet werden, um das Reinigungsmedium entsprechend aufzuwärmen. Hierbei ist es dann nicht mehr oder kaum noch erforderlich, dass das Heizelement 10 Wärme erzeugt. Zusätzlich ist es mithilfe des Lüfters 11 möglich, dass, für den Fall, dass zu viel Wärme gespeichert ist, eine entsprechende Abkühlung erfolgt, wodurch immer erreicht wird, dass das Reinigungsmedium im vorgegebenen Temperaturbereich den Instrumenten 4 zugeführt wird.

## Patentansprüche

1. Gerät (1) zum Reinigen, Desinfizieren, Sterilisieren und/oder Pflegen von ärztlichen, insbesondere zahnärztlichen Instrumenten (4), aufweisend
● eine Pflege- und Reinigungskammer (2),
● in der Pflege- und Reinigungskammer (2) befindliche Halterungen (3) zur Aufnahme der Instrumente (4) und
● Mittel (7, 12, 14) zum Zuführen von Reinigungs- oder Pflegemedien zu den Instrumenten (4),
**dadurch gekennzeichnet,**
**dass** das Gerät (1) eine Vorrichtung (9) zur Temperierung aufweist, die ein Reinigungsmedium, das durch die Mittel (7, 12, 14) zum Zuführen den Instrumenten (4) zugeführt wird, auf einen vorgegebenen Temperaturbereich temperieren kann.

2. Gerät nach Anspruch 1,
**dadurch gekennzeichnet,**
**dass** das Gerät (1) einen Kühl-/Heizkreislauf (8) aufweist, der mit den Halterungen (3) zur Aufnahme der Instrumente (4) in Verbindung steht und mit den Mitteln (7, 12, 14) zum Zuführen thermisch gekoppelt ist.

3. Gerät nach Anspruch 2,
**dadurch gekennzeichnet,**
**dass** der Kühl-/Heizkreislauf (8) mit der Vorrichtung (9) zur Temperierung in Verbindung steht und mit den Mitteln (7, 12, 14) zum Zuführen in der Vorrichtung (9) zur Temperierung thermisch gekoppelt ist.

4. Gerät nach Anspruch 3,
**dadurch gekennzeichnet,**
**dass** der Kühl-/Heizkreislauf (8) und die Mittel (7, 12, 14) zum Zuführen durch einen in der Vorrichtung (9) zur Temperierung angeordneten Metallblock thermisch gekoppelt sind.

5. Gerät nach einem vorherigen Ansprüche,
**dadurch gekennzeichnet,**
**dass** die Vorrichtung (9) zur Temperierung Mittel zum Heizen (10) aufweist.

6. Gerät nach einem vorherigen Ansprüche,
**dadurch gekennzeichnet,**
**dass** die Vorrichtung (9) zur Temperierung Mittel zum Kühlen (11) aufweist.

7. Gerät nach einem vorherigen Ansprüche,
**dadurch gekennzeichnet,**
**dass** der vorgegebene Temperaturbereich 40°-60°C ist.

8. Gerät nach einem vorherigen Ansprüche,
**dadurch gekennzeichnet,**
**dass** die Halterungen (3) zur Aufnahme der Instrumente (4) jeweils aus einem Magnetventilblock (6) und einem Kupplungsadapter (5) bestehen, wobei die Instrumente (4) an den Kupplungsadaptern (5) angebracht werden.

9. Gerät nach einem vorherigen Ansprüche,
**dadurch gekennzeichnet,**
**dass** die Mittel (7, 12, 14) zum Zuführen eine Pumpe (12) aufweisen.

10. Gerät nach einem vorherigen Ansprüche,
**dadurch gekennzeichnet,**
**dass** in dem Kühl-/Heizkreislauf (8) eine Pumpe (13) angeordnet ist.

11. Verfahren zum Reinigen, Desinfizieren, Sterilisieren und/oder Pflegen von ärztlichen, insbesondere zahnärztlichen Instrumenten (4), bei dem die aufzubereitenden Instrumente (4) an Halterungen (3) in einer Pflege- und Reinigungskammer (2) eines Geräts (1) zum Reinigen, Desinfizieren, Sterilisieren und/oder Pflegen von ärztlichen, insbesondere zahnärztlichen Instrumenten (4), angeordnet werden und bei dem den Instrumenten (4) ein Reinigungsmedium zur Reinigung zugeführt wird,
**dadurch gekennzeichnet,**
**dass** das Reinigungsmedium auf einen vorgegebenen Temperaturbereich temperiert wird.

12. Verfahren nach Anspruch 11,
**dadurch gekennzeichnet,**
**dass** durch einen Kühl-/Heizkreislauf (8) die Halterungen (3) zur Aufnahme der Instrumente (4) gekühlt bzw. erwärmt werden und dass bei Kühlung der Halterungen (3) die in den Kühl-/Heizkreislauf (8) abgegebene Wärme zur Erwärmung des Reinigungsmediums verwendet wird.

13. Verfahren nach einem der Ansprüche 11-12,
**dadurch gekennzeichnet,**
**dass** das Reinigungsmedium erwärmt wird.

14. Verfahren nach einem der Ansprüche 11-13,
**dadurch gekennzeichnet,**
**dass** das Reinigungsmedium gekühlt wird.

15. Verfahren nach einem der Ansprüche 11-14,
**dadurch gekennzeichnet,**
**dass** der vorgegebene Temperaturbereich 40°-60°C ist.
